# EUROPEAN PATENT APPLICATION

(11) **EP 0 829 268 A2**
(43) Date of publication of application: **18.03.1998**
(21) Application number: 97307158.2
(22) Date of filing: 15.09.1997
(51) Int. Cl.: A61M 5/24, A61M 5/34

(54) **Non-floatable insulin cartridge holder for medication delivery pen**

(30) Priority: 17.09.1996 US 714904
(71) Applicant: Becton, Dickinson and Company, Franklin Lakes, New Jersey 07417-1880 (US)
(72) Inventor: Bush, Charles L., Jr., Fairfeld, New Jersey 07004 (US); Paddock, Douglas, Hamburg, New Jersey 07419 (US)
(74) Representative: Ruffles, Graham Keith

(57) **Abstract**

A two piece insulin cartridge holder is provided for a medication delivery pen. The two piece insulin cartridge holder includes a generally tubular body for receiving, supporting and accurately positioning the body and shoulder portions of a cartridge of medication. A needle mounting insert tip is snap fit and non-floatably mounted to the body of the insulin cartridge holder for receiving the neck, rubber septum and crimped metallic sleeve of the cartridge.

## Description

### BACKGROUND OF THE INVENTION

### 1. Field of the Invention.

The subject invention relates to the portion of a medication delivery pen that retains the cartridge of medication and, more particularly, a non-floatable cartridge holder.

### 2. Background Description.

Medication delivery pens are hypodermic syringes that are used for self-injection of precisely measured doses of medication. Pens are widely used, for example, by diabetics to dispense insulin.

A typical prior art medication delivery pen includes a cartridge which contains a volume of liquid medication sufficient for several doses. The cartridge includes an elongated generally tubular glass cartridge having a pierceable rubber septum which extends across the open distal end of the cartridge and is securely held in position by a metallic sleeve that is crimped to the distal end of the cartridge. The cartridge also includes a rubber stopper in sliding fluid-tight engagement with interior walls of the cartridge.

Such a medication delivery pen also includes a unitarily molded cartridge retainer having a small diameter tubular neck dimensioned for tightly engaging the neck of the cartridge and the metallic sleeve crimped thereon so as to support and position the entire cartridge. Exterior regions at the extreme distal end of the tubular neck are formed with an array of threads for threadedly receiving the mounting cap of a needle assembly. The medication delivery pen further includes a dosing apparatus that is engaged with the proximal end of the cartridge retainer having a plunger for engaging the rubber stopper of the cartridge. The dosing apparatus includes a dose setting structure used to select the longitudinal distance through which the plunger will move, and dispensing means for driving the plunger the selected distance.

The needle assembly for the medication delivery pen includes an elongate needle cannula having opposed proximal and distal points and a lumen extending therethrough. A plastic cork is adhered to an intermediate position along the needle cannula and in turn is rigidly connected to an end wall of a cylindrical cap. The cylindrical wall of the cap surrounds the proximal point on the needle cannula and includes an array of internal threads for engaging the external threads on the neck of the cartridge retainer.

The medication delivery pen may be used by urging the cap of the needle assembly over the neck of the cartridge retainer sufficiently for the proximal point of the needle cannula to pierce the rubber septum of the cartridge. The cap is then rotated to threadedly engage the neck of the cartridge retainer. The user then manipulates the dosing apparatus to select an appropriate dose. A protective shield over the distal end of the needle cannula is then removed, and the distal point of the needle cannula is injected. The user then actuates the dispensing means of the prior art dosing apparatus to urge the stopper of the cartridge distally and to deliver medication through the lumen of the needle cannula. The needle is then withdrawn, and the needle assembly is separated from the cartridge retainer and safely discarded. The rubber septum of the cartridge reseals itself, and may be pierced again for a subsequent administration of medication. This process may be carried out repeatedly until all of the medication in the cartridge has been used.

Since cartridges are subject to a considerable range of dimensional variations and a considerable degree of eccentricity, it is often difficult to precisely form a cartridge retainer to closely engage, support and position the entire cartridge. These dimensional variations and eccentricities may be due to the glass cartridge manufacturing processes or to the crimping of the metallic sleeve that holds the rubber septum in place. Dimensional variations can result in a cartridge that will not fit the cartridge retainer or that will be loosely supported and movable therein. Eccentricities can result in a cartridge barrel that is not properly positioned or aligned within the body of the cartridge retainer. Eccentricities also can prevent the neck of the cartridge from sliding into the precisely dimensioned neck of the cartridge retainer. As a result, considerable quality control efforts must be undertaken to ensure that only cartridges that are within narrowly defined dimensional tolerances are used, and high reject rates occur. To reduce rejects and ensure that a larger number of cartridges can be accepted, prior art pens have included cartridge retainers with wider bodies that are intended to accommodate a greater range of eccentricities between the neck and the body of the cartridge. This results in larger pens even though it would be desirable to reduce the size.

### SUMMARY OF THE INVENTION

The subject invention is directed to a two-piece cartridge retainer assembly that is particularly suitable for medication delivery pens. The cartridge retainer assembly is made of two materials including a generally tubular body made of plastic surrounding the barrel of the cartridge and for supportingly engaging the converging wall that defines the shoulder of the cartridge and a metal needle mounting insert tip non-floatably mounted to the body for surrounding the neck of the cartridge. The needle mounting insert tip includes a rim extending from its proximal end that is diametrically dimensioned to closely engage the metallic sleeve which is crimped to the cartridge for holding the rubber septum in place. The needle mounting insert tip of the cartridge retainer assembly also includes an axial length for preventing contact between the rubber septum and the cap or cork of the needle assembly and is made from metal to provide the strength required to sustain greater impact during the injection process.

The needle mounting insert tip is held by four locking tabs on the cartridge retainer that lock into place in four locking pockets on the insert tip. This snap fit allows for a one time assembly of each part and if the insert tip is manipulated to attempt removal from the cartridge retainer, the cartridge retainer will be destroyed and can no longer be used. The major advantage over prior art devices that are 100% plastic is the strength and flexibility of the cartridge retainer. The metal insert tip sustains a greater impact and still is able to function where as plastic tip will "stress crack" and possibly render the parts useless. In addition, a snap fit insert tip provides for various revisions to be made to the cartridge retainer as an assembly without reworking or manufacturing several different cartridge retainer molds. Different insert tips can be manufactured and snapped into the same cartridge retainer body.

These and other aspects, features and advantages of the present invention will become apparent from the following detailed description taken in conjunction with the accompanying drawings.

### BRIEF DESCRIPTION OF THE DRAWINGS

Fig. 1 is an exploded perspective view of a medication delivery pen having a cartridge retainer assembly in accordance with the subject invention;
Fig. 2 is an exploded perspective view of the cartridge retainer assembly of the subject invention; and
Fig. 3 is a cross-sectional view of the cartridge retainer assembly.

### DETAILED DESCRIPTION OF THE PREFERRED EMBODIMENT

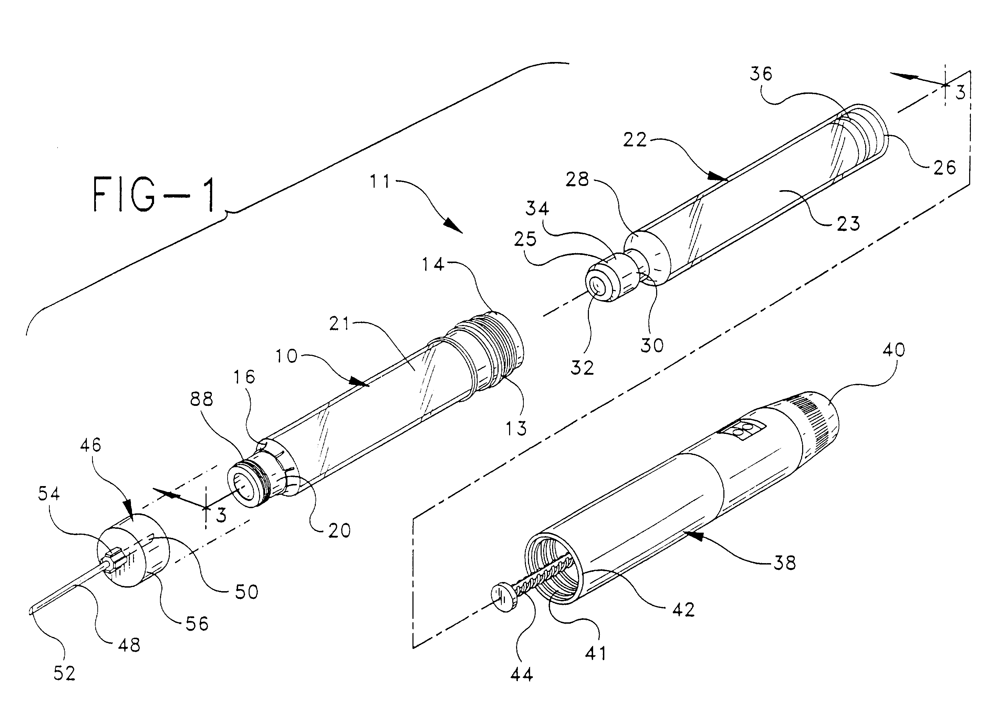

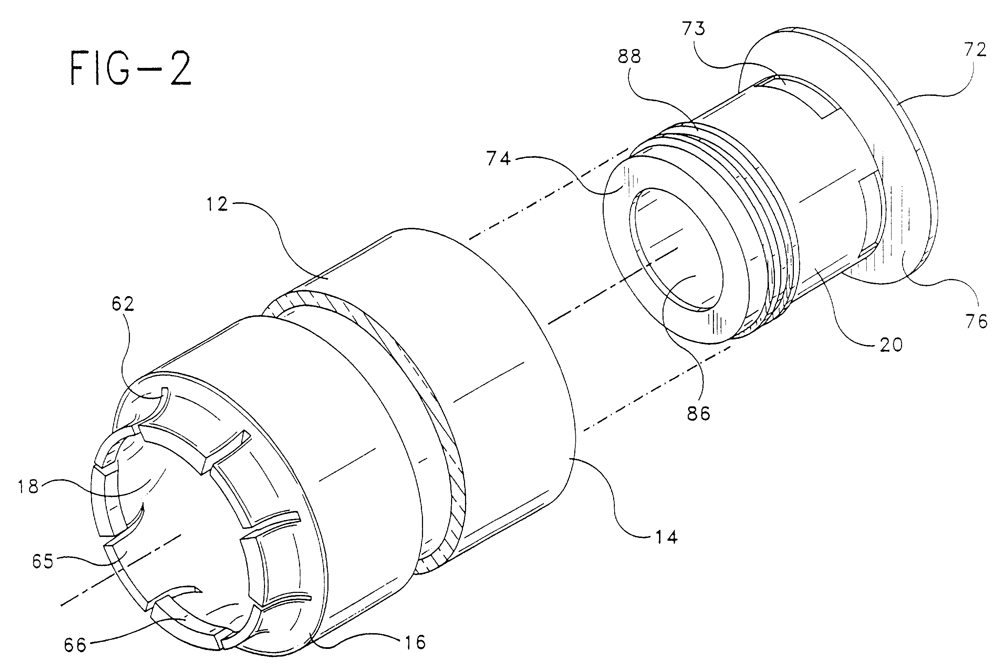

A cartridge retainer assembly in accordance with the subject invention is identified generally by the numeral 10 in Fig. 1. Cartridge retainer assembly 10 is intended to be a part of a medication delivery pen 11 and includes elongate generally tubular body 12 with opposed proximal and distal ends 14 and 16, respectively, and a cartridge receiving cavity 18 (shown in Fig. 2) extending therebetween. A generally tubular needle mounting insert tip 20 is snap-fit mounted in distal end 16 of body 12. Body 12 and insert tip 20 of cartridge retainer assembly 10 both are described in greater detail below.

Cartridge retainer assembly 10 is dimensioned and configured to receive a cartridge assembly 22 therein. Cartridge assembly 22 includes a cartridge 23 with a generally tubular barrel 24 with an open proximal end 26 and a distal end 25 defined by an inwardly converging shoulder 28. A small diameter neck 30 projects distally from shoulder 28 of barrel 24 on cartridge 23, and is provided with a large diameter annular bead 24 extending circumferentially thereabout at the extreme distal end of neck 30. A pierceable and resealable rubber septum 32 extends completely across the open distal end defined by neck 30. Rubber septum 32 is held in place by a metallic sleeve 34 which is crimped around the circumferential bead at the distal end of neck 30. Medication such as insulin or heparin is pre-filled into cartridge 23 and is retained therein by a rubber stopper 36. Stopper 36 is in sliding fluid-tight engagement with the tubular wall of barrel 24. Distally directed forces on stopper 36 urge the medication from pen 11 as explained further below.

Medication delivery pen 11 further includes a prior art dosing apparatus identified generally by the numeral 38. Dosing apparatus 38 is generally cylindrical and includes opposed proximal and distal ends 40 and 42, respectively. Threads 41 are disposed at distal end 42 of dosing apparatus 38 for releasable threaded engagement with proximal end 14 of body 12 of cartridge retainer assembly 10. A plunger rod 44 projects distally from dosing apparatus 38 and is dimensioned to engage stopper 36 of cartridge assembly 22. The dosing apparatus 38 includes known mechanisms therein for setting a selected dose of medication to be delivered by pen 11. A dispensing mechanism (not shown) is operative to drive plunger rod 44 a selected distance in a distal direction for moving stopper 36 a distance that will inject the selected dose of medication from cartridge assembly 22. Although a particular prior art dosing apparatus 38 is depicted in Fig. 1, it is to be understood that other dosing apparatus can be used with the cartridge retainer assembly of the subject invention.

Medication delivery pen 11 further includes a needle assembly 46 having a metallic needle cannula 48 with opposed proximal and distal tips 50 and 52, respectively, and a lumen (not shown) extending entirely therethrough. A cork 54 is securely affixed at an intermediate position along needle cannula 48, and a cap 56 is securely affixed to cork 54. Cap 56 of needle assembly 46 includes an array of internal threads (not shown) for removable mounting needle assembly 46 to cartridge retainer assembly 10.

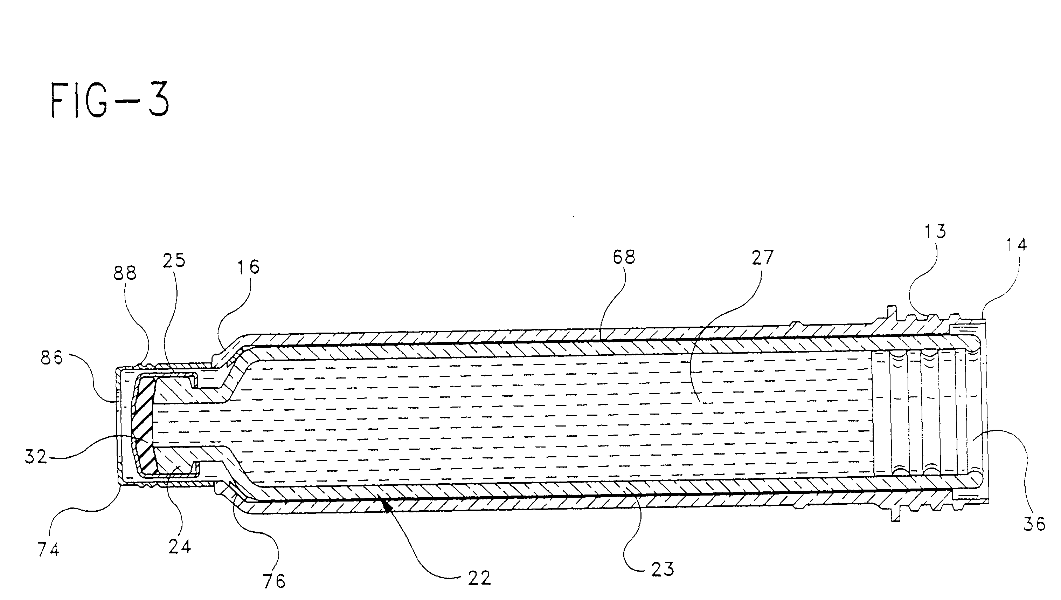

As explained above, prior art cartridge assemblies 22 are subject to significant dimensional variation and eccentricities. In particular, the crimped mounting of metal sleeve 34 to neck 30 can result in diametrical or axially length differences from one cartridge to the next. Additionally, considerable eccentricities between neck 30 and body 24 are likely to exist. Cartridge retainer assembly 10 accommodates the dimensional variations and eccentricities that exist in prior art cartridge assemblies 22. Fig. 2 is an exploded perspective view and Fig. 3 is a cross-sectional view of cartridge retainer assembly 10, which together show the features of the present invention that overcome these problems. More particularly, as shown in Fig. 2, body 12 of cartridge retainer assembly 10 includes a plurality of inwardly projecting supports 65 defining sections of arcs concentric with body 12. Supports 65 are separated from one another by notches 62. Every other support 65 includes a locking tab 66 at its extreme end that mate with locking pockets 73 on insert tip 20.

Portions of body 12 disposed proximally of supports 65 define a cartridge receiving chamber 68 of substantially uniform diameter which is slightly greater than diameter of cartridge barrel 24. Proximal end 14 of body 12 includes an array of threads 13 that engage threads on proximal end 42 of dosing apparatus 38. It is to be understood, however, that other releasable engagement means between dosing apparatus 38 and cartridge retainer assembly 10 can be provided. For example, external threads can be formed on dosing apparatus 38 and corresponding internal threads can be defined on cartridge retainer assembly 10 or a bayonet style mounting using lugs and slots can be used.

Needle mounting insert tip 20 of cartridge retainer assembly 10 includes opposed proximal and distal ends 72 and 74, respectively, as shown in Fig. 2. Proximal end 72 is characterized by a plurality of locking pockets 73 dimensioned and disposed to receive each locking tab 66 on arcuate supports 65 of cartridge retainer body 12.

Proximal end 72 of needle mounting insert tip 20 includes a rim 76 extending therefrom that is diametrically dimensioned to closely engage metallic sleeve 34 crimped to cartridge 23 for holding rubber septum 32 in place. Distal end 74 of needle mounting insert tip 20 includes a generally annular end wall 84 having an aperture 86 extending therethrough for access bv proximal point 50 of needle cannula 48. An array of outwardly disposed threads 88 is defined intermediate proximal and distal ends 72 and 74, respectively. Threads 88 are disposed and dimensioned for engaging threads on prior art needle assembly 46.

Needle mounting insert tip 20 and cartridge retainer body 12 are lockingly engaged with one another prior to sale of pen 11 by urging distal end 74 of insert tip 20 into proximal end 14 of body 12. This assembly is carried out by first aligning locking pockets 73 at proximal end 72 of insert tip 20 with locking tabs 66 on supports 65 at distal end 16 of body 12. After sufficient movement of insert tip 20 and body 12 toward one another, each locking pocket 73 receives and mates with a respective locking tab 66 and firmly and non-floatably locks insert tip 20 on distal end 16 of body 12. As shown in Fig. 2, needle mounting insert tip 20 is held by four locking tabs 66 on cartridge retainer body 12 that lock into place in four locking pockets 73 on insert tip 20. This snap fit allows for a one time assembly of each part and if insert tip 20 is manipulated to attempt removal from cartridge retainer body 12, cartridge retainer body 12 will be destroyed and can no longer be used. The major advantage over prior art devices that are 100% plastic is the strength and flexibility of the cartridge retainer. The metal insert tip 20 sustains a greater impact and still is able to function where as plastic tip will "stress crack" and possibly render the parts useless. In addition, a snap fit insert tip provides for various revisions to be made to cartridge retainer 10 as an assembly without reworking or manufacturing several different cartridge retainer molds. Different insert tips 20 can be manufactured and snapped into the same cartridge retainer body 12.

Assembly of medication delivery pen 11 continues by inserting cartridge 22 into cartridge retainer assembly 10. More particularly, neck 30 and crimped metallic sleeve 34 of cartridge 22 are inserted in a proximal to distal direction into open proximal end 14 of body 12 of cartridge retainer assembly 10. Crimped metallic sleeve 34 eventually will pass entirely through body 12, and further advancement of cartridge 22 into cartridge retainer assembly 10 will require entry of crimped metallic sleeve 34 into rim 76 extending from proximal end 72 of needle mounting insert tip 20. As noted above, considerable dimensional variation and eccentricities between the neck and body of prior art cartridges are known to exist. If such eccentricities do exist, crimped metallic sleeve 34 will rest on rim 76 of insert tip 20 to center sleeve 34 relative to body 12 into a position that conforms with any dimensional inconsistencies or eccentricities in cartridge 22.

Further distally directed movement of cartridge 22 into cartridge retainer assembly 10 will cause shoulder 28 of cartridge 22 to seat against rim 76 of insert tip 20. Rim 76 therefore defines the fully seated position of cartridge 22 in cartridge retainer assembly 10 and functions to securely engage cartridge 22. In this fully seated position, as shown most clearly in Fig. 3, septum 32 of cartridge 22 is spaced proximally from distal wall 84 of needle mounting insert tip 20.

Dosing apparatus 38 may next be assembled to proximal end 14 of the body of cartridge retainer assembly 10 such that plunger rod 44 of dosing apparatus 38 engages stopper 26 of cartridge 22.

Medication delivery pen 11 may be used by mounting a needle assembly 46 to needle mounting insert tip 20 of cartridge retainer assembly 10. This mounting is achieved by moving needle assembly 46 in a proximal direction over needle mounting insert tip 20 until the threads (not shown) of cap 56 engage external threads 88 on needle mounting insert tip 20. As noted above, threads 88 of needle mounting insert tip 20 are spaced from the extreme distal end of needle mounting insert tip 20. Thus, the initial axial advancement of cap 56 over needle mounting insert tip 20 will cause proximal point 50 of needle cannula 48 to pierce rubber septum 32 of cartridge 22 prior to rotational threaded engagement of needle assembly 46 with needle mounting insert tip 20. Thus, the bevel which defines proximal point 50 will advance axially through septum 32 without a rotation that could tear rubber septum 32. After threads of cap 56 engage threads 88 of needle mounting insert tip 20, further advancement of needle assembly 46 requires relative rotation between cap 56 and needle mounting insert tip 20. It will be appreciated that needle mounting insert tip 20 is too small to be readily griped by the user of medication delivery pen 11, and is partly covered by cap 56. However, the relative rotation can be achieved by rotating body 12 of cartridge retainer assembly 10. In particular, as noted above, locking pockets 73 of needle mounting insert tip 20 are engaged by locking tabs 66 on body 12. Hence, rotation of body 12 is transmitted to needle mounting insert tip 20 and enables complete rotational engagement of needle assembly 46.

Use of medication delivery pen 11 proceeds in a conventional manner with dosing apparatus 38. As explained above, actuation of dosing apparatus 10 causes liquid medication in cartridge 22 to be urged in a distal direction. The medication will be urged through the lumen of needle cannula 48. This distally directed liquid pressure also will cause septum 32 to distend in a distal direction. However, as noted above and as shown in Fig. 3, septum 32 is spaced proximally from cork 54 of needle assembly 46, and will not be urged into contact with cork 54. Thus, drooling or weeping of liquid medication can be substantially prevented. This is enabled because cartridge 22 is supported and accurately positioned by engagement of cartridge shoulder 28 with rim 76 on insert tip 20. Hence neck 30 and crimped metallic sleeve 34 need not be closely engaged by needle mounting insert tip 20.

After medication delivery pen 11 has been used, needle assembly 46 is separated from needle mounting insert tip 20 and discarded. The user is encouraged to apply a disinfectant to the distal end of medication delivery pen 11. Disinfectants have the potential of adversely affecting some plastic materials that could be used in a medication delivery pen. However, the two-part construction of cartridge retainer assembly 10 has a metal needle mounting insert tip 20 that is resistant to disinfectants that may be applied by the user. Cartridge retainer body 12 is made of Lexan^{TM} which is a clear plastic material manufactured by GE Plastics and is processed into the cartridge retainer form by an injection molding process. Insert tip 20 is made of aluminum and is formed using a combination stamping and machining process.

In the foregoing discussion, it is to be understood that the above-described embodiments of the present invention are simply illustrative of various features of a cartridge retainer assembly for a medication delivery pen. Other suitable variations, modifications and combinations of these features could be made to or used in these embodiments and still remain within the scope of the present invention.

## Claims

1. A cartridge retainer assembly for retaining a medication cartridge, said cartridge having a barrel, a neck defining a smaller cross-section than said barrel and a converging shoulder extending therebetween, said cartridge retainer assembly comprising:
a generally tubular body having opposed proximal and distal ends and a chamber therebetween, said chamber being dimensioned and configured for engaging said barrel of said cartridge therein, said body including a plurality of inwardly projecting supports defining a distal end of said chamber and including at least one locking tab formed on one of said supports; and
a generally tubular needle mounting insert tip having opposed proximal and distal ends, said proximal end of said insert tip including a locking pocket for receiving said at least one locking tab on said body to prevent floating, rotation and axial movement between said needle mounting insert tip and said body.

2. The cartridge retainer assembly of Claim 1, wherein said needle mounting insert tip includes a rim that mates with the neck of said cartridge to center said cartridge in said body.

3. The cartridge retainer assembly of Claim 1, wherein:
said body includes eight of said supports with four of said supports including said locking tabs; and
said needle mounting tip includes four of said locking pockets for mating with said four locking tabs.

4. The cartridge retainer assembly of Claim 1, wherein said needle mounting insert tip and said body are formed from dissimilar materials.

5. The cartridge retainer assembly of Claim 1, wherein said needle mounting insert tip comprises an array of external threads thereon for threadedly and releasably engaging a needle assembly, said threads on said needle mounting insert tip being disposed proximally of said distal end of said needle mounting insert tip.

6. The cartridge retainer assembly of Claim 1, wherein:
said body is formed from a transparent plastic material; and
said needle mounting insert tip is formed from a metallic material.

7. The cartridge retainer assembly of Claim 1, wherein:
said body is formed from a transparent plastic material; and
said needle mounting insert tip is formed from aluminum.

8. The cartridge retainer assembly of Claim 1, wherein said needle mounting insert tip defines a length sufficient to extend distally beyond the neck of said cartridge when said shoulder and said barrel are engaged in said body of said cartridge retainer assembly.
